# EUROPEAN PATENT SPECIFICATION

(11) **EP 1 991 548 B1**
(45) Date of publication and mention of the grant of the patent: **11.12.2013**
(21) Application number: 07712727.2
(22) Date of filing: 19.02.2007
(51) Int. Cl.: C07D 487/04, A61K 31/5025, A61P 19/00, A61P 17/00, A61P 3/00

(54) **Salt of CD80 antagonist**
Salz eines CD80-Antagonisten
Sel d'un antagoniste de CD80

(30) Priority: 22.02.2006 GB 0603522
(43) Date of publication of application: 19.11.2008
(73) Proprietor: Medigene AG, 82152 Planegg/Martinsried (DE)
(72) Inventor: MATTHEWS, Ian, Richard, Berkshire RG40 2LG (GB)
(74) Representative: Bösl, Raphael Konrad
(86) International application number: PCT/GB2007/000550
(87) International publication number: WO 2007/096588

(56) References cited:
- WO-A1-2004/081011
- C. HAYNES ET AL.: "Occurrence of pharmaceutically acceptable anions and cations in the Cambridge structural database.", JOURNAL OF PHARMACEUTICAL SCIENCES, vol. 94, no. 10 , - 2005,

## Description

The present invention relates to the choline salt of the CD80 antagonist compound 4-(6-fluoro-3-oxo-1,3-dihydro-pyrazolo[4,3-c]cinnolin-2-yl)-N-(2,2-difluoro-ethyl)-benzamide.

### Background to the invention

International patent application No. WO 2004/081011 relates to a class of compounds including (*inter alia*) the compound 4-(6-fluoro-3-oxo-1,3-dihydro-pyrazolo[4,3-c]cinnolin-2-yl)-N-(2,2-difluoro-ethyl)-benzamide, having the structural formula (A):

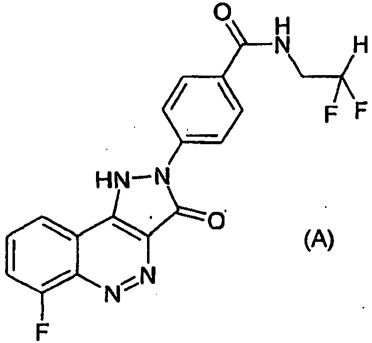

The compounds disclosed in WO 2004/081011 are CD80 antagonists, capable of inhibiting the interactions between CD80 and CD28, and therefore useful for immuno-inhibition, for example in the treatment of rheumatoid arthritis. The compound (A), in the form of the free acid, is poorly soluble in water. In general good water solubility is a desirable characteristic in a compound intended for oral administration, or parenteral or topical administration in a water based carrier, as a pharmaceutical product. WO 2004/081011 also refers to salts of the compound class of which compound (A) is a member. However, not all salts of the compound have sufficiently improved aqueous solubility over the free acid to be convenient orally administrable, or aqueous solution-based, forms of the compound.

### Brief Description of the Invention

This invention is based on the finding that the choline salt of compound (A) has the desired good aqueous solubility.

### Detailed Description of the invention

According to the present invention, there is provided the choline salt of 4-(6-fluoro-3-oxo-1 ,3-d ihyd ro-pyrazolo[4, 3-c]cin nolin-2-yl)-N-(2,2-difl uoro-ethyl)-benzamide of formula (A) above.

Orally administrable pharmaceutical compositions comprising the said choline salt and at least one pharmaceutically acceptable carrier also form an aspect of the invention.

Injectable aqueous solutions of the said choline salt, and topically applicable aqueous based liquid or cream compositions containing the said choline salt form another aspect of the invention.

The orally administrable compositions may be in the form of tablets, capsules, pellets, powders, granules, lozenges, liquid or gel preparations. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The active ingredient may also be administered parenterally in a sterile medium, by injection or infusion. Depending on the vehicle and concentration used, the drug can either be suspended or dissolved in the vehicle. Advantageously, adjuvants such as a local anaesthetic, preservative and buffering agents can be dissolved in the vehicle.

The preparation of, and aqueous solubility of, the choline salt of the invention are further described in the following Example.

### Example:

### 4-(6-Fluoro-3-oxo-1,3-dihydro-pyrazolo[4,3-c]cinnolin-2-yl)-N-(2,2-difluoro-eth yl)-benzamide was prepared and its choline (ie (2-hydroxy-ethyl)-trimethylammonium) salt formed, as follows:

### Preparation of N-(2,2-Difluoro-ethyl)-4-(6-fluoro-3-oxo-1,3-dihydro-pyrazolo[4,3-c]cinnolin-2-yl)-benzamide.

A round bottom flask equipped with a magnetic stirrer, reflux condenser and gas bubbler was charged with 4-(6-Fluoro-3-oxo-1,3-dihydro-pyrazolo[4,3-c]cinnolin-2-yl)-benzoic acid (12.9 g) prepared as in Example 5 of WO 2004/081011. Thionyl chloride (65 ml) was added slowly. A nitrogen atmosphere was applied and the mixture was heated to reflux. Upon heating gas evolution was observed, the gas was trapped in a scrubber. When the gas evolution had ceased (after approx. 2 h) the mixture had changed colour from an orange-red suspension to a dark red suspension and was cooled to room temperature. Excess thionyl chloride was removed under vacuum and the obtained red solid was azeotroped with toluene (50 ml). A dark red solid was obtained and taken up in anhydrous DMA (65 ml) to give a dark red solution. Diisopropyl ethyl amine (12.9 g, 17.4 ml) was mixed with 2,2-difluoroaminoethane (3.24 g, 2.76 ml) and added drop-wise to the solution over 4-5 minutes. An exothermic was observed. The mixture was stirred at room temperature.

The reaction mixture was quenched with 0.5 M HCl (150 ml) to give a dark red suspension. The solids were collected by filtration and washed with some water. The solid was triturated with methanol (250 ml) for approx. 1 hour at room temperature, filtered and washed with methanol. The solid was then triturated with acetone (250 ml) for approx. 1 hour at room temperature, filtered and washed with acetone. The solid was finally triturated with ethyl acetate (250 ml) for approx. 45 minutes, filtered and washed with ethyl acetate.

The product (approx. 8 g) was taken up in approx. 10 ml of DMA to give a thick dark red solution. Methanol (150 ml) and acetone (150 ml) were added and the precipitate collected by filtration.
LC-MS: One peak of required product.. QC analysis of this material showed a purity of 91.6%.

### Preparation of 2-[4-(2,2-Difluoro-ethylcarbamoyl)-phenyl]-6-fluoro-2H-pyrazolo[4,3-c]cinnolin-3-olate (2-hydroxy-ethyl)-trimethyl-ammonium

A round bottom flask fitted with a magnetic stirrer was charged with N-(2,2-Difluoro-ethyl)-4-(6-fluoro-3-oxo-1,3-dihydro-pyrazolo[4,3-c]cinnolin-2-yl)-benzamide (2.50 g). The solid was suspended in methanol (25 ml) and choline hydroxide (782 mg, 1.74 ml 45% w/w in methanol) was added drop-wise. Upon addition a clear dark brown solution was formed. The solution was stirred for 2 hours at room temperature. The reaction mixture was concentrated under vacuum to give a dark brown oil. Ethyl acetate (100 ml) was added and a two-phase system was obtained. After sonication the brown oil became thick and started to solidify. The solid was broken up and isopropyl alcohol (30 ml) was added and the mixture was heated to 65-70°C. A suspension was obtained. The mixture was cooled to room temperature and diluted with ethyl acetate (approx. 100 ml). The solids were collected by filtration and washed with ethyl acetate (50 ml). The solid was dried under vacuum.
LC-MS: isolated solid; one main peak [M+H]+ 388, purity 95%.

The solid salt was a brown coarse powder, 2.26 g, 4.61 mmol, 71 % Melting point: 172-180°C, decomposition.

The product was further purified by recrystallisation, as follows: The salt was mixed with ethanol and heated to reflux, stirred for 20 minutes, and filtered hot. The filtered ethanol solution was heated to reflux, then cooled to about 5deg C and stirred for one hour. The solution was then filtered, washed with ethanol and heptane, and dried to constant weight.

The solubility of the choline salt of N-(2,2-difluoro-ethyl)-4-(6-fluoro-3-oxo-1,3-dihydro-pyrazolo[4,3-c]cinnolin-2-yl)-benzamide in water and ethanol was tested in the following assay. The solubilities of the non-salt form (free acid) N-(2,2-difluoro-ethyl)-4-(6-fluoro-3-oxo-1,3-dihydro-pyrazolo[4,3-c]cinnolin-2-yl)-benzamide and a range of other salts thereof were also tested in the same assay:

### Solubility Assay

10 mg of the test compound was weighed into a vial, and 1 ml of solvent was added. If the sample dissolved immediately, as evidenced by a formation of a clear solution, more of the test material was added and the vial sonicated for 15 mins. This procedure was repeated until no more test compound could be dissolved, as evidenced by formation of a suspension. The vial containing the suspension was then sonicated for 15 mins, and placed on a shaker at 25 deg C/60% relative humidity for 24 hours. On removal from the shaker, the sample was centrifuged and the supernatant analysed by HPLC. The results are given in Table I.

| Test Salt | Solubility (mg/ml) in water | Solubility (mg/ml) in ethanol |
|---|---|---|
| None (free acid) | <0.5 | 2.1 |
| Choline | >51 | >21 |
| Potassium | <5 | <5 |
| Sodium | <5 | 7.1 |
| Magnesium | <5 | <5 |
| Lysine | <5 | <5 |
| Arginine | <5 | <5 |

## Claims

1. The choline salt of 4-(6-fluoro-3-oxo-1,3-dihydro-pyrazolo[4,3-c]cinnolin-2-yl)-N-(2,2-difluoro-ethyl)-benzamide of formula (A):

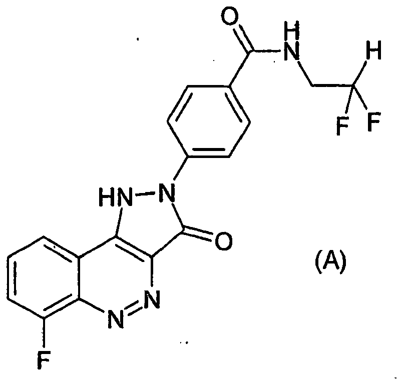

2. An orally administrable pharmaceutical composition containing the choline salt claimed in claim 1 and at least one pharmaceutically acceptable carrier.

3. An injectable aqueous solution of the said choline salt claimed in claim 1.

4. A topically applicable aqueous based liquid or cream composition containing the choline salt claimed in claim 1.

## Patentansprüche

1. Das Cholin-Salz von 4-(6-Fluor-3-oxo-1,3-dihydro-pyrazolo[4,3-c]cinnolin-2-yl)-N-(2,2-difluor-ethyl)-benzamid der Formel (A):

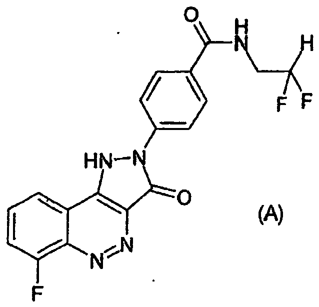

2. Oral verabreichbare pharmazeutische Zusammensetzung enthaltend das in Anspruch 1 beanspruchte Cholin-Salz und mindestens einen pharmazeutisch verträglichen Träger.

3. Injizierbare wässrige Lösung des in Anspruch 1 beanspruchten Cholin-Salzes.

4. Topisch anwendbare wasserbasierte Zusammensetzung einer Flüssigkeit oder einer Creme enthaltend das in Anspruch 1 beanspruchte Cholin-Salz.

## Revendications

1. Sel de choline de 4-(6-fluoro-3-oxo-1,3-dihydro-pyrazolo[4,3-c]cinnolin-2-yl)-N-(2,2-difluoro-éthyl)-benzamide de formule (A) :

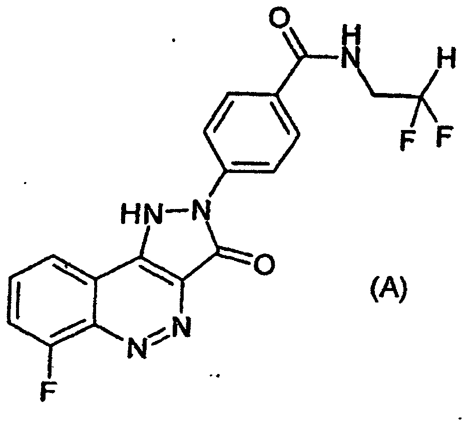

2. Composition pharmaceutique administrable par voie orale contenant le sel de choline selon la revendication 1 et au moins un support pharmaceutiquement acceptable.

3. Solution aqueuse injectable dudit sel de choline selon la revendication 1.

4. Liquide à base aqueuse ou composition crémeuse applicable de manière topique contenant le sel de choline selon la revendication 1.
